# EUROPEAN PATENT SPECIFICATION

(11) **EP 3 588 119 B1**
(45) Date of publication and mention of the grant of the patent: **03.03.2021**
(21) Application number: 18179817.4
(22) Date of filing: 26.06.2018
(51) Int. Cl.: G01R 33/30, A61B 5/055, G01R 33/567, A61N 5/10

(54) **HEAD COIL ARRANGEMENT FOR A MAGNETIC RESONANCE DEVICE WITH IMPROVED IMMOBILIZATION**
KOPFSPULENANORDNUNG FÜR MAGNETRESONANZGERÄT MIT VERBESSERTER IMMOBILISIERUNG
AGENCEMENT DE BOBINE DE TÊTE POUR APPAREIL DE RÉSONANCE MAGNÉTIQUE PRÉSENTANT UNE MEILLEURE IMMOBILISATION

(43) Date of publication of application: 01.01.2020
(73) Proprietor: Medical Intelligence Medizintechnik GmbH, 86830 Schwabmünchen (DE)
(72) Inventor: LIU, Rui, 86153 Augsburg (DE)
(74) Representative: Maiwald Patent- und Rechtsanwaltsgesellschaft mbH

(56) References cited:
- WO-A1-2018/094538
- US-A- 5 954 647
- US-A1- 2009 048 508
- US-A1- 2011 230 755
- US-A1- 2017 065 830
- US-B2- 8 190 237
- V. MENON ET AL: "Design and efficacy of a head-coil bite bar for reducing movement-related artifacts during functional MRI scanning", BEHAVIOR RESEARCH METHODS, INSTRUMENTS AND COMPUTERS, vol. 29, no. 4, 1 December 1997 (1997-12-01), pages 589-594, XP055525942, US ISSN: 0743-3808, DOI: 10.3758/BF03210613
- SPICHER N. ET AL: "High-speed, contact-free measurement of the photoplethysmography waveform for MRI triggering", PROCEEDINGS OF THE INTERNATIONAL SOCIETY FOR MAGNETIC RESONANCE IN MEDICINE, 24TH ANNUAL MEETING AND EXHIBITION, SINGAPORE, 7-13 MAY 2016, vol. 24, 22 April 2016 (2016-04-22), page 1861, XP040682903,
- HANVEY S ET AL: "Magnetic resonance imaging for radiotherapy planning of brain cancer patients using immobilization and surface coils; Magnetic resonance imaging for radiotherapy planning of brain cancer patients", PHYSICS IN MEDICINE AND BIOLOGY, INSTITUTE OF PHYSICS PUBLISHING, BRISTOL GB, vol. 54, no. 18, 21 September 2009 (2009-09-21), pages 5381-5394, XP020163629, ISSN: 0031-9155, DOI: 10.1088/0031-9155/54/18/002

## Description

### Field of the invention

The present invention relates generally to magnetic resonance image (MRI)-guided radiation therapy. In particular, the present invention relates to a magnetic resonance (MR) head coil arrangement, a magnetic resonance device and a method of immobilizing, providing improved immobilization of a patient.

### Background of the invention

Medical imaging is commonly used to assist in the diagnosis and/or treatment of patients. Magnetic resonance imaging (MRI) is an example of a medical imaging technology that is often performed during the diagnosis and treatment of tumors. To this end, the MRI may be performed at the same time as diagnosing and irradiating the tumor, wherein radiation may be performed by e.g. a linear accelerator (LINAC)-system.

In order to be able to irradiate the tumor in the best possible way without compromising healthy tissue surrounding the tumor, high quality MR images are sought. For this, MRI may use local radio-frequency coils (RF coils) as a receiver and/or transmitter which, for example, increase a SNR (signal-to-noise ratio) to obtain better quality images. These RF coils may be carried by a magnetic resonance coil arrangement. Such a local RF coil may be arranged in the area of a patient's head and can therefore referred to as an MR head coil. For example, EP 1 113 287 A2 and US 8 190 237 B2 disclose such an MR head coil.

Additionally, when using MRI for diagnosis and/or treatment, it is generally desired that the patient moves as little as possible. One advantage is that this generally results in higher quality images during imaging since, for example, blurry artifacts within the images can be avoided. In this regard, the longer the image acquisition time is, the more important it is that the patient moves as little as possible. Equally, during irradiation, e.g. during MR-LINAC treatment, the patient should move as little as possible, since any unexpected movement may lead to an unsafe situation. Such an unsafe situation may be that unintentionally healthy tissue is irradiated.

Therefore, in the prior art, several position detecting methods have been proposed in order to detect patient movement. For example, US 6 259 942 B1 discloses a mouthpiece used as a reference device for being detected by a positional detector. Further, US 2017/0032538 A1 proposes a computer-implemented method for determining false motion tracked by a motion correction system during a medical imaging scan, wherein one or more markers are placed one or more portions of the patient. Similarly, US 2017/0065830 A1 discloses a head coil arrangement provided with a plurality of fiducial markers that are configured for emitting magnetic resonance signals, wherein these markers are imaged and their position is evaluated and the position of the head coil is determined.

Further, in order to avoid or at least reduce movements of the patient, several fixation means have been proposed. For example, US 8 099 150 B2 discloses a head support assembly configured to be removably secured to an MRI scanner gantry or treatment table, wherein a plurality of head engagement rods adjustably associated with the head support frame are configured to engage the patient's skull. Further, US 2015/0119902 A1 proposes arranging a frame around the patient's head and screwing fixation pins connected to the frame into a bone of the skull, which requires an invasive or surgical procedure. Another proposal is directed to a net-like mask which is placed over the patient's head and attached to a treatment table. These proposals have in common the disadvantage that fixation may be uncomfortable for the patient due to the invasive contact with the skull. Also, the set up time required for positioning and fixing the patient's head may be too high. Further, when inserting screws into the patient's skull, it may interfere with the radiation beam during treatment.

US 2009/0048508 A1 describes an attachment apparatus for optimising the attachment position of a stereotactic base ring to a subject's head. The attachment apparatus comprises one or more fixing elements that allow the attachment apparatus to be releasably attached to a stereotactic base ring. V. MENON ET AL: "Design and efficacy of a head-coil bite bar for reducing movement-rel ated artifacts during functional MRI scanning", BEHAVIOR RESEARCH METHODS,

INSTRUMENTS AND COMPUTERS, vol. 29, no. 4, 1 December 1997, describes the design, construction, application, and effectiveness of a simple bite bar for use with an MRI whole-head coil to reduce head-movement-related artifacts during functional brain imaging. Therefore, it remains a need to provide a sufficient fixation and positioning of the patient during imaging and/or radiation treatment of tumors which should be as comfortable as possible for the patient.

### Summary of the invention

An aspect of the present invention provides a head coil arrangement as defined in independent claim 1 for a magnetic resonance (MR) device with a helmet-shaped coil housing comprising at least one conductor carrier extending in a distance to a head supporting plane and forming in between a receiving space for a patient's head. The head coil arrangement further comprises a head-fixation structure, which is supported, via at least one leg, at the conductor carrier so as to at least partially protrude into the receiving space and configured to be brought into blunt contact with the patient.

In this context, under the helmet-shaped coil housing may be understood that a forehead, a face, eyes, a nose, cheeks, a mouth and/or a chin is at least partly surrounded by the coil housing. Accordingly, the receiving space may be enclosed. Preferably, the coil housing may be configured like a full-face helmet. The head-fixation structure may preferably be configured to be brought into blunt contact with the patient, which in particular includes non-surgical contact and/or non-invasive contact (i.e. where healthy tissue is not penetrated or injured). In particular, there is no engagement with a cranial bone, e.g. the skull, of the patient. Further, a conductor, which may be formed as a coil wire, can be arranged within the conductor carrier.

Several advantages may be achieved with this configuration of the head coil arrangement. On the one hand, the coil housing required anyway for carrying the RF coil fulfills a dual function, namely additionally as a carrier for the head-fixation structure. Thereby, head movement of the patient may be reduced. On the other hand, the noninvasive contact with the patient reduces set-up time and increases comfort for the patient, especially for patients tending to claustrophobia. Further, by supporting the head-fixation structure at the coil housing, these two components form a structural unit, further reducing set-up time by being able to be arranged and moved together.

In an embodiment of the invention, the frame structure may further comprise at least one first conductor carrier and at least one second conductor carrier which together span a conductor plane at least partially surrounding the receiving space. In other words, the coil housing may carry several conductors which form a RF head coil carried by the coil housing, wherein the RF head coil may be configured as a multi-channel RF head coil. Also, the conductors may span the patient's head when arranged in the receiving space.

Thus, the head coil arrangement provides both a good signal-to-noise ratio and an improved immobilization of the patient.

According to a further embodiment of the invention, the head-fixation structure is made from an electromagnetically inert material. The material may be a composite and/or plastic material, such as epoxy resin, polyester, polyacrylamide, vinyl ester resin, wood, ceramic, aramid, glass fiber, polyethylene, etc.

Thus, MRI is not impaired by the head-fixation structure arranged between the patient's head and the RF coil and/or the radiation source. In particular, blurry artefacts within the images may be avoided.

According to the invention, the head-fixation structure i is molded in one piece with the coil housing. In other words, the head-fixation structure and the coil housing may be formed integrally by the same material. Likewise, the leg arranged between the head-fixation structure and coil housing may be molded in one piece with both the coil housing and the head-fixation structure.

Thus, the head coil arrangement can be provided as a compact structural unit.

In one embodiment of the invention, the head-fixation structure may comprise a bite-lock mouthpiece. This mouthpiece may be personalized for an individual patient. As explained above, the mouthpiece is formed integrally with the coil housing. Also, a blunt contact is ensured, as it is taken in the mouth and when used properly neither penetrates nor damages a mouth mucosa.

Thus, the patient's head can be fixed by simply taking the mouthpiece into the mouth. As a result, the eyes and the face remain free which is more comfortable for patients with a tendency to claustrophobia. In particular, an oral cavity of the patient can be immobilized. In addition, a vacuum can be generated by a pump fluidically connected to the mouthpiece in order to fix the patient's mandible.

According to a further embodiment of the invention, the head-fixation structure may comprise a chinrest. With respect to a patient's chin, the chinrest may be formed concave shaped, in particular like a basket, to encompass the chin from the outside. For example, the chinrest may be arranged so as to push the chin against a head supporting surface, e.g. which may be a patient support table or a head supporting part of the coil housing, from a direction facing away from the head supporting surface.

Thus, due to the blunt surface contact with the chin, the head-fixation structure impairs the patient even less and thus provides more comfort. In particular, the majority of the patient's face is not in contact with a restraining surface which is beneficial for claustrophobic patients.

In a further embodiment of the invention, the head-fixation structure may comprise a frontal bone holder. For example, the frontal bone holder may be arranged so as to abut on and push the frontal bone against a head supporting surface, e.g. which may be a patient support table or a head supporting part of the coil housing, from a direction facing away from the head supporting surface.

Thus, due to the blunt surface contact with the frontal bone, the head-fixation structure impairs the patient even less and thus provides more comfort. In particular, the majority of the patient's face is not in contact with a restraining surface which is beneficial for claustrophobic patients.

Is should be noted that, in another embodiment of the invention, the above mouthpiece, the chinrest and the frontal bone holder may be combined within the head-fixation structure.

Thus, the patient's head may be fixed on at least three different positions which both provides effective restriction of the possible movements of the patient's head, and, provides the patient with the comfort of a less restricted view..

According to a further embodiment of the invention, both the head-fixation structure and the coil housing may comprise at least one fiducial marker arranged so as to be trackable within a MR image. By way of example, the fiducial markers may be infrared (IR) markers or the like. Other embodiments of the fiducial marker will be described below.

Thus, any movement of the patient can be detected. Also, movement of the head-fixation structure relative to the coil housing can be detected. This can significantly increase safety because e.g. the radiation source can be disabled during an undesired movement.

In a further embodiment of the invention, the coil housing may comprise at least one first sensor configured to measure a signal propagation time for determining a position of the patient relative to the coil housing. The first sensor may be utilized as the above coil-housing-sided fiducial marker, wherein no external IR light is used in this case. Further, the first sensor may be configured as a time-of-flight sensor, e.g. a time-of-flight camera system, measuring the direct time-of-flight required for a single light pulse (e.g., photon) to leave the first sensor and reflect back onto e.g. a focal plane array of the first sensor. In other words, e.g. infrared signals are sent and received by the first sensor, wherein the time difference between sending and receiving can be multiplied by the speed of light to determine the distance between the patient and the coil housing. The first sensor may be embedded in the material of the coil housing due to its small package size. The first sensor may be utilized for different tasks during MRI and/or treatment. For example, the position or an image of the patient's head and/or face may be captured by the first sensor. A data processing device, which may be a part of a MR device, may be configured for face recognition based on the data provided by the first sensor. Further, the coil housing may also comprise several, e.g. two, three, four or more, first sensors, which may be connected to each other and/or to a data processing device so as to computationally construct a multi-dimensional model of the patient's head and/or face. For different measuring tasks, the first sensors may have different characteristics, such as a high resolution and lower speed, a lower resolution and higher speed, or the like.

Thus, the first sensor may capture three-dimensional images and can contain complete spatial and temporal data, imaging full three-dimensional scenes with a single light pulse. Also, eye motion of the patient can be captured. Compared with X-ray or MR images, constructing a multi-dimensional model by the first sensor configured as a time-of-flight sensor is noninvasive as e.g. IR light is utilized. In addition, the first sensor provides fast imaging, e.g. less than milliseconds, a high resolution, e.g. sub-mm scale, and is low energy. Furthermore, the first sensor is mechanically and electrically robust and is available at low cost.

In addition, the first sensor may be used for setting up the patient by measuring a distance between the patient's skin and the RF coil. This measured distance may be used as a trigger for sending a stop interlock signal to a LINAC and/or the MR scanner.

The first sensor may further be used for a quality check and/or for registration of the head-fixation structure, e.g. the above mouthpiece, before MRI and/or treatment. In this regard, the head-fixation structure may be optically detected by the first sensor, wherein a 3D model may be created, and may be compared with reference data assigned to the individual head-fixation structure. The reference data can consist of a reference 3D model that is stored in a patient database. Thus, performance degradation and/or assignment to a wrong patient of the head-fixation structure may be avoided.

Further, the first sensor may be used to create a 3D model of the patient's head which may be combined with 3D model data related to the one or more coils accommodated within the coil housing as well with patient's 3D skeletal anatomy data, obtained e.g. from a CT scanner during treatment dose calculation. These combined data may be used to determine a specific absorption rate (SAR) in order to achieve an optimal trade-off between SAR, MR imaging quality and patient's head position. This trade-off may be done with finite element method (FEM) and/or an electromagnetic (EM) simulator. Thus, exceeding predetermined SAR and thus an increasing temperature within the patient can be avoided.

According to a further embodiment, the coil housing may comprise at least one second sensor configured to measure a light absorption or the light remission on transillumination of a skin of the patient. The first sensor may be utilized as the above coil-housing-sided fiducial marker, wherein no IR light is used in this case. Further, the second sensor may be configured as a e.g. photoplethysmography (PPG) sensor which, basically, illuminates the patient's skin and measures changes in light absorption. Accordingly, the second sensor may provide a contactless imaging method to collect in-vivo signals of the patient. Further, data of the second sensor may be combined with data of the first sensor for patient identification purposes, e.g. for face recognition, wherein the second sensor may provide skin properties of the patient.

Thus, by use of the second sensor, skin properties, heart and breathing rate, mental stress and/or sleep patterns etc. of the patient can be measured. On basis of such information, also treatment or shut down in case of an emergency or the like can be controlled.

In another embodiment, the head coil arrangement may further comprise at least one acoustic transducer arranged at the head-fixation structure and configured to send an acoustic signal via one or more cranial bones to an internal ear of the patient. The acoustic transducer may be an electromechanical actuator that utilizes bone conduction, wherein sound is conducted to the inner ear through cranial bones and/or bones of the skull.

Thus, no headphones are needed to provide communication from a clinician to the patient, and the patient can wear ear protection, e.g. ear plugs or the like. As a result, the patient can still hear the sound instruction from clinicians while less space is needed around the patient's ears. In addition, humans can typically hear sound when the frequency of acoustic signals lies between 20 Hz and 20 kHz. Accordingly, a preferred operating frequency of the acoustic transducer lies between 20 Hz and 20 kHz. Therefore, the operating frequency is significantly below a typical MR signal frequency used in MRI, being e.g. around 64 MHz for a 1.5 T MR scanner. Thus, no MRI artifacts will be generated. Furthermore, mechanical vibrations generated by the acoustic transducer are small enough not to adversely affect the head-fixation structure.

Another aspect of the invention provides a magnetic resonance (MR) device comprising a MR scanner and head coil arrangement according to one or more of the above-explained embodiments. The MR scanner may comprise a bore and a gantry or patient support table.

This MR device improves immobilization of the patient due to the head-fixation structure of the head coil arrangement.

In an embodiment of the invention, the MR device may further comprise a data processing device configured to create a multi-dimensional model of a patient's head on basis of data provided by one or more sensors arranged at the head coil arrangement. These sensors may be e.g. the above time-of-flight sensor, the above PPG sensor etc.

Thus, the model may be used through a whole clinical workflow. For example, the model may be used to reduce the patient's set-up time since the model provides information indicating a preferred head location and/or safety distance within an MR device.

According to an embodiment of the invention, the model comprises three geometric dimensions extended by a fourth time-related dimension.

Thus, the 4D model may be used as a prime imaging guidance for radiotherapy, thereby reducing an invasive demand of X-ray and/or MR images.

In another embodiment of the invention, the MR device may further comprise a synchronizing device communicatively connected to both the head coil arrangement and the magnetic resonance scanner and configured to temporarily put an electrical component of one of the magnetic resonance scanner and the head coil arrangement in a different operating state during a specific task is performed with the other one of the magnetic resonance scanner and the head coil arrangement. The synchronizing device may be an electronic device.

Thus, MRI artifacts caused by an electric and/or electronic device can be avoided since these can be disabled, by e.g. shut off power supply or the like.

According to an embodiment of the invention, the synchronizing device may be configured to temporarily change a light emitted by a light source arranged in a bore of the MR scanner while controlling a measurement via one or more optical sensors arranged at the head coil arrangement.

Thus, the light within the bore can be changed to a color less sensitive to the IR light of the second sensor. For example, the light within the bore can be changed to a bluish spectrum not affecting the optical measurement of the second sensor. This can also be done only for a few milliseconds, the patient hardly or not at all aware of this change.

In another embodiment of the invention, the synchronizing device is configured to temporarily disable a power supply of one or more optical sensors arranged at the head coil arrangement during imaging by the magnetic resonance scanner.

Thus, the synchronizing device can temporarily power off the first and/or second sensor during MR imaging, during which e.g. an MRI signal receiver and/or an MRI signal readout is active. In this way, the first sensor and/or second sensor is disabled and the head coil arrangement temporarily becomes a conventional head coil without generating MRI artifacts. According to an embodiment of the invention, the synchronizing device is configured to temporarily disable a power supply of the acoustic transducer of the head coil arrangement during imaging by the magnetic resonance scanner.

Thus, electronic noise of the acoustic transducer can be suppressed. In this way, the acoustic communication is only disabled for a short time, during which no MRI artifacts are generated.

An aspect of the invention provides a method of immobilizing a head within a MR device as defined in independent claim 18.

The method may be performed by the above head coil arrangement and/or the MR device.

The method comprises:
- providing a helmet-shaped coil housing comprising at least one conductor carrier extending in a distance to a head supporting plane and forming in between a receiving space for a patient's head,
- providing a head-fixation structure, which is supported, via at least one leg, at the conductor carrier so as to at least partially protrude into the receiving space and configured to be brought into noninvasive contact with the patient,
- wherein a movement relative to the coil housing and/or the head-fixation structure is monitored by utilizing one or more markers and/or one or more sensors arranged at the coil housing and/or the head-fixation structure.

Thus, the method improves immobilizing a patient's head during MR imaging and/or treatment. In particular, the head-fixation structure is supported by the coil housing and forms one single structural unit.

According to an embodiment of the invention, the method further comprises:
- scanning a surface of the patient's head by utilizing the one or more sensors,
- creating a multi-dimensional model of the patient's head on basis of data obtained by the scanning, and
- verifying, on the basis of the model, if the position of the patient's head is correct.

Thus, the coil housing and/or the head-fixation structure carries technical equipment for monitoring the patient's head position.

Alternatively or in addition, both the head-fixation structure and the coil housing comprise at least one fiducial marker arranged so as to be trackable within a magnetic resonance image, so the position of MR head coil arrangement can be determined and further mapped with the surface scanning of the patient's head and anatomy data inside of patient's brain.

Thus, monitoring of the patient's head position can be further improved.

In another embodiment of the invention, on the basis of the model, real-time treatment dose planning is performed.

Thus, a beam shape can be generated so as to not go through e.g. the coil conductors or any electronics arranged within the receiving space and/or the coil housing of the head coil arrangement. In this way, such electronics are less impacted by radiation, and inducing radiation activity can be avoided.

It should be noted that embodiments as described above may be combined with respect to each other so as to gain a synergetic effect, which may extend over the separate technical effects of the single features. Likewise, the above method can be modified by the embodiments of the above head coil arrangement and/or the magnetic resonance device and vice versa.

### Brief description of the figures

Exemplary embodiments of the invention will be described in the following with reference to the following figures.
- Figure 1: illustrates an MR device having a support table and an MR head coil arrangement which is arranged on it, according to an example outside the scope of the invention.
- Figure 2: shows an MR head coil arrangement according to an embodiment of the invention.
- Figure 3: illustrates an MR head coil arrangement according to a further embodiment of the invention.
- Figure 4: shows an MR head coil arrangement according to a further embodiment of the invention.
- Figure 5: shows an MR head coil arrangement according to a further embodiment of the invention.
- Figure 6: illustrates an MR head coil arrangement according to a further embodiment of the invention.
- Figure 7: shows a block diagram of an MR device according to an embodiment of the invention.
- Figure 8: shows a principle creating of a model by use of a time-of-flight sensor arranged at an MR head coil arrangement according to an embodiment of the invention.
- Figure 9: shows a flowchart of a method for setting up and/or immobilizing a patient within an MR device, according to an embodiment of the invention.

The figures are merely schematic representations and serve only to illustrate the invention. Identical or equivalent elements are consistently provided with the same reference signs.

### Detailed description of exemplary embodiments

In the following, a detailed description of exemplary embodiments will be given to explain the invention in more detail.

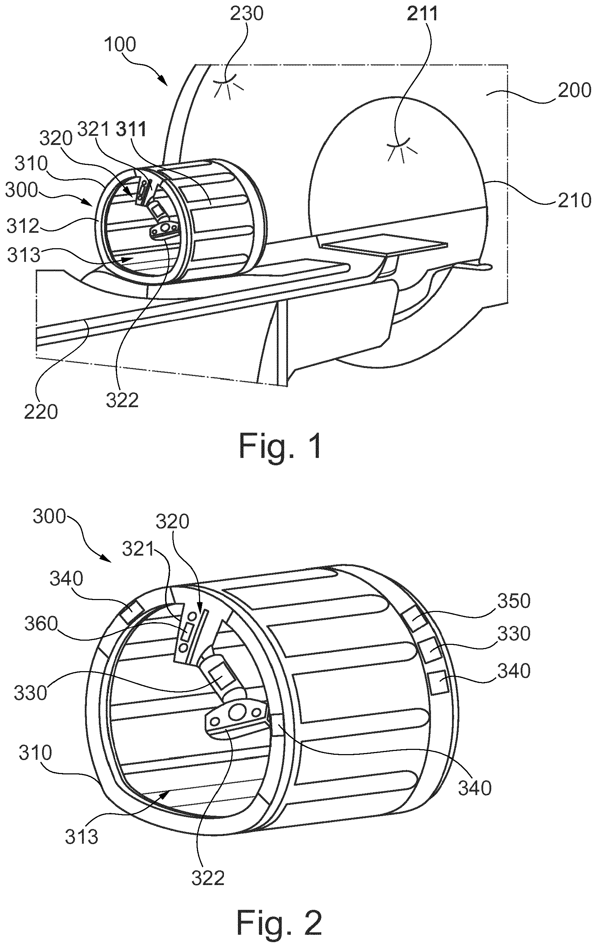

Figure 1 illustrates schematically an MR device 100 which comprises an MR scanner 200 with a bore 210, a patient support table 220 standing on a floor surface and an MR head coil arrangement 300 arranged on the support table 220. Within the bore 210, a first light source 211 emitting visible light is arranged. A second light source 230 also emitting visible light is arranged outside the bore 210 on a side wall or ceiling.

A top surface of the support table 220 defines a lying plane LP for a recumbent patient (not shown) and can optionally be moved in and out the bore 210 of the MR scanner 200. The MR head coil arrangement 300 is placed on the top surface of the support table 220 and comprises a helmet-shaped coil housing 310 with a plurality of first conductor carriers 311 and a plurality of second conductor carriers 312. The first conductor carriers 311 extend parallel to a longitudinal axis of the coil housing 310, which longitudinal axis is parallel to that of the support table 220. The second conductor carriers 312 extend circumferentially around the longitudinal axis. The coil housing 310 is made from an electromagnetically inert material, preferably from a suitable plastic and/or composite material. The conductor carriers 311, 312 extend in a distance to the lying plane LP, which also supports a head of the patient, and form in between a receiving space 313 for the patient's head. Further, the conductor carriers 311, 312 each carry an electrical wire (not shown) which together form an RF coil 314 for receiving RF signals, wherein the conductor carriers 311, 312 together span a conductor plane that partially surrounds the receiving space 313.

The head coil arrangement 300 comprises a head-fixation structure 320 for fixing the patient head within the receiving space 313. By way of example, the head-fixation structure 320 is supported via leg 321 at the second conductor carrier 312. Both the head-fixation structure 320 and the leg 321 are made from an electromagnetically inert material, preferably from a suitable plastic and/or composite material. In this exemplarily embodiment, the head-fixation structure 320 and the leg 321 are formed integrally, i.e. in one piece, with the coil housing 310 so that the materials are the same. It should be noted that the head-fixation structure 320 and the leg 321 are adjustable relative to the patient, i.e. to the receiving space. Further, the head-fixation structure 320 protrudes into the receiving space 313 and is configured to be brought into noninvasive contact with the patient. Several positions for noninvasive contacting the patient and respective embodiments and examples outside the scope of the present invention of the head-fixation structure 320 will be described below. According to this example, the head-fixation structure 320 comprises a bite-lock mouthpiece 322 which is releasably attached to the leg 321 so that it can be exchanged and the coil housing 310 is reusable with another patient.

Figure 2 shows an embodiment of the head coil arrangement 300 as a single component. It comprises at least two fiducial markers 330 which are visible and/or trackable within MR images. At least one of the fiducial markers 330 is arranged at the coil housing 310 and at least another one is arranged at the head-fixation structure 320. The head coil arrangement 300 further comprises a plurality of first sensors 340, which in this embodiment are designed as time-of-flight-sensors, e.g. as on-chip sensors. Each of the first sensors 340 is configured to measure a signal propagation time between the first sensor 340 and the patient, e.g. for determining a position of the patient relative to the coil housing 310 and/or setting up the MR device 100 as well as the patient etc., as described below. The first sensors 340 are embedded within the material of the coil housing 310, distributed around and aligned towards the receiving space 313. Further, the head coil arrangement 300 comprises at least one second sensor 350, which in this embodiment is designed as a photoplethysmogram (PPG) sensor, configured to measure a light absorption or the light remission on transillumination of a skin of the patient. Accordingly, the second sensor 350 is configured to measure and/or monitor heart rate, cardiac cycle, respiration, hypo- and hyperkalemia etc. of the patient. Also, the second sensor 350 is embedded within the material of the coil housing 310 and aligned towards the receiving space 313. For enabling communication between a clinician and the patient, the head coil arrangement 300 comprises an acoustic transducer 360 arranged at the head-fixation structure 320 and configured to convert electric or, as preferred here, optical signals into mechanical vibrations and sending sound to the internal ear through cranial bones of the patient. It should be noted that the first sensors 340, the second sensor 350 and the acoustic transducer 360 are connected to a power source and at least partially communicatively connected to a data processing device 110, as described below. Merely as an example, the head-fixation structure 320 of this embodiments comprises the bite-lock mouthpiece 322 which can be replaced by another fixation means.

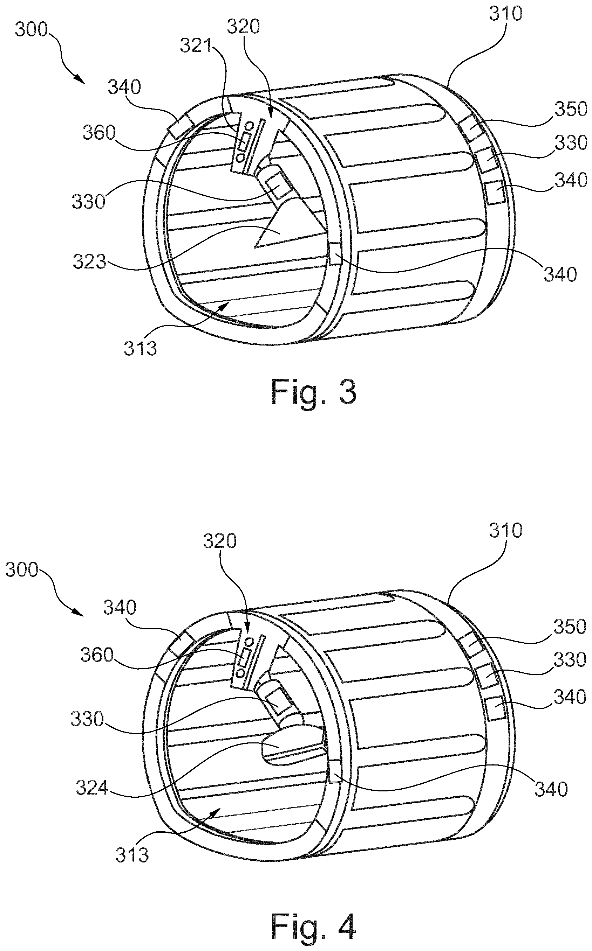

In Figure 3, a further embodiment of the head coil arrangement 300 is shown. In principle, this is the same as that described above. However, in this embodiment, the head-fixation structure 320 has another fixation means, namely a chinrest 323. The chinrest 323 is also attached to the leg 321 and is cup-shaped, thereby configured to at least partially enclose the chin of the patient. Also, the chinrest 323 is configured to be moved toward the patient's chin relative to the coil housing 310.

Figure 4 shows a further embodiment of the head coil arrangement 300, which differs from the above embodiments mainly in the design of the fixation means. Accordingly, the head-fixation structure 320 of this embodiment comprises a frontal bone holder 324 that is configured to be brought into contact with the frontal bone of the patient. The frontal bone holder is bar-shaped and adapted to the shape of the frontal bone. Also, the frontal bone holder 324 is configured to be moved toward the patient's chin relative to the coil housing 310.

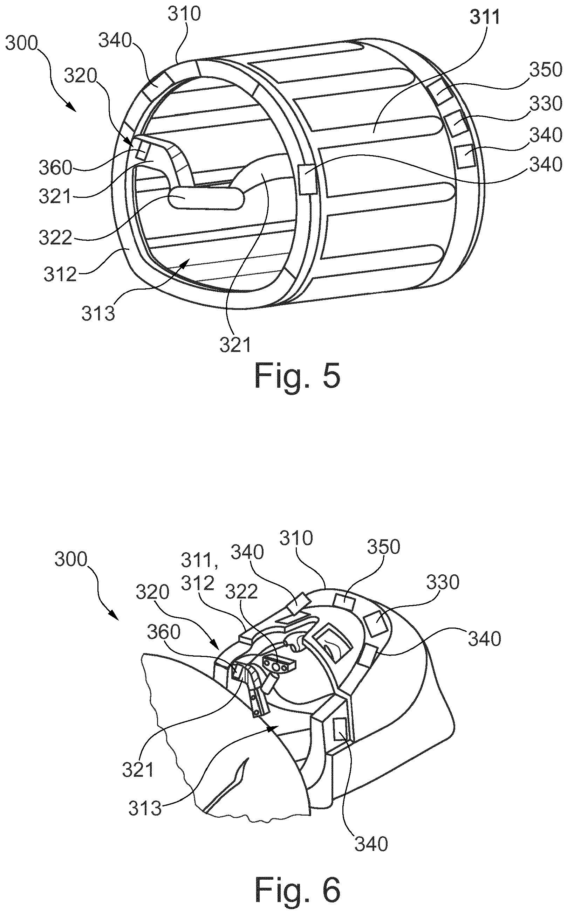

In Figure 5, a further embodiment of the head coil arrangement 300 is shown. This embodiment differs from that above in that a second leg 321 extends away from the coil housing 310. By way of example, the first and second leg 321 are arranged symmetrically with respect to the head-fixation structure 320, which may be configured as the mouthpiece 322, the chinrest 323 or the frontal bone holder 324.

Figure 6 shows a further embodiment of the head coil arrangement 300. It differs from the embodiments above mainly in the design of the conductor carriers 311, 312, i.e. in their number, shape, and their extension direction. This embodiment has in common with the above that the head-fixation structure 320 is supported, via the leg 321, at the coil housing 310.

Also, the head-fixation structure 320 is designed as explained above. It should be noted that the coil housing 310 may be configured in two parts with a lower part and an optionally attachable and detachable upper part. In this embodiment, the head-fixation structure 320 is attached to the upper part of the coil housing 310.

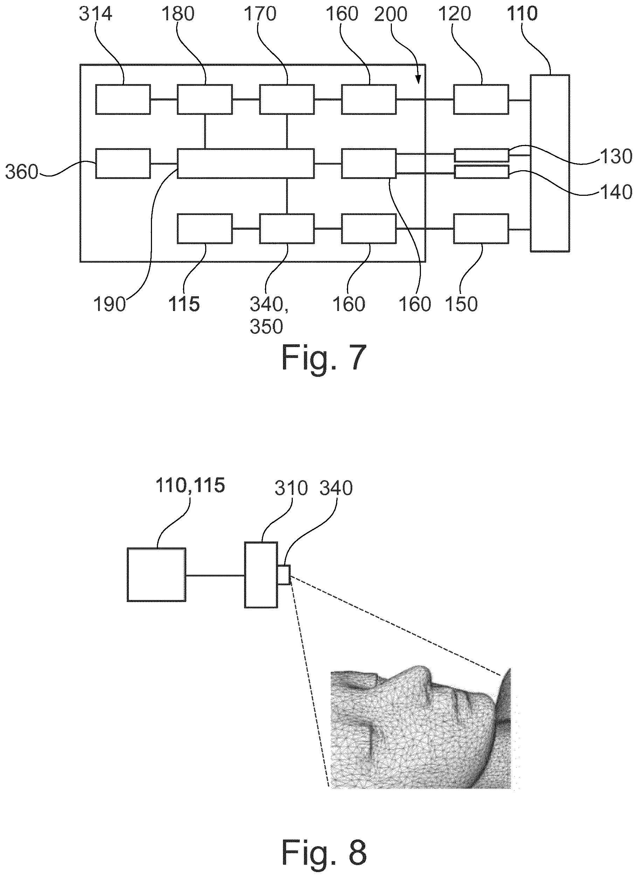

With reference to Figure 7, which shows a block diagram, the electric and/or electronic structure of the MR device 100 will now be described. Accordingly, the MR device 100 comprises the central data processing device 110 as mentioned above, which is configured to process data of the MR device 100 and to control its main functions, e.g. by a processor, an I/O-interface etc. The data processing device 110 is connected to a MRI post processing unit 120, a gradient amplifier 130, configured to provide output voltages and currents and to control gradient coils (not shown), a light controlling unit 140 and a LINAC interface 150. These components have in common that they are arranged in a room separated to the MR scanner 200 (see also Figure 1), which is indicated in Figure 7 by a border surrounding the further components assigned to the MR scanner 200 or the room accommodating it. Within that room, the MR device 100 further comprises a plurality of optical converters 160, which are each configured to convert electrical signals used outside the room accommodating the MR scanner 200 into optical signals used inside that room. A first one of the optical converters 160 is connected to the MRI post-processing unit 120 outside the room, and connected inside the room to an MRI signal readout unit 170, which is also connected to an MRI signal receiver 180 and to the RF coil 314. A second one of the optical converters 160 is connected to the LINAC interface 150 outside the room, and connected to the first and second sensors 340, 350, inside the room, wherein these sensors 340, 350 are also connected to a storage unit 115, configured as e.g. a flash drive to store data of the sensors 340, 350 and/or the data processing device 110. A third one of the optical converters 160 is connected to the gradient amplifier 130 and the light controlling unit 140 and outside the room and connected to a synchronizing device 190 inside the room. It should be noted that this synchronizing device 190 is also connected to the second one of the optical converters 160 connected to the LINAC interface 150, the sensors 340, 350, the MRI signal readout unit 170, the MRI signal receiver 180 and the acoustic transducer 360.

Still referring to Figure 7, the functions of the above components as well as an exemplary operation of the MR device 100 will be described in the following.

First, the head-fixation structure 320 may be used to immobilize the patient's head during MRI and/or treatment within the MR device 100. For this purpose, the patient lies down on the support table 220 with his head placed inside the receiving space 313 of the head coil arrangement 300 surrounded by the RF coil 314. The patient's head is then fixed by use of the head-fixation structure 320, wherein at least one of the bite-lock mouthpiece 322, the chinrest 323 and the frontal bone holder 324 is brought into noninvasive contact with the patient's mouth, chin or frontal bone to immobilize the head relative to the coil housing 310 and the RF coil 314.

Next, the first sensor 340 may be used for several tasks before and/or during MRI and/or treatment. Accordingly, after the patient's set up within the head coil arrangement 300, the first sensor 340 is used to capture the patient's face and/or head outline. In addition, the second sensor 350 captures a skin property of the patient. Then, data of the first sensor 340 and the second sensor 350 are combined and processed by e.g. the data processing device 110 for patient identification. For this purpose, the data processing device 110 comprises a software module configured to derive face recognition from image recognition. Further, the first sensor 340 is used to detect and/or register the head-fixation structure 320, and in particular the mouthpiece 322 which is personalized for the patient. In this regard, one or more of the first sensors 340 3D scan e.g. the mouthpiece 322 and, for example, the data processing device 110 analyses the data of the first sensor 340 by comparing it with a prescanned 3D model obtained by a patient database. As a result, it is verified whether it is the right mouthpiece 322 as assigned to the patient and whether this is still usable in qualitative terms. Furthermore, by use of the first sensor 340, a distance between the patient's skin and the RF coil 314 is measured. The measured distance is recorded to the storage unit 115 as a distance threshold. In case the distance between the RF coil 314 and the patient's skin is out of a pre-defined range and may cause unexpected radiation dose increment due to, for example, ERE (electron return effect), the first sensor 340 can send a radiation beam stop interlock signal to the LINAC interface 150 via the respective optical converter 160. Due to the distance threshold already stored in the storage unit 115, no MR images have to be taken or processed by the MRI post processing unit 120 in order to determine the distance between the patient's skin and the RF coil. Compared to measuring the distance using MRI, this approach requires less time and is more reliable to avoid unexpected radiation dose increment. In addition, the first sensors 340 provides real-time distance measuring which is used in an online-radiation dose planning module (not shown). Further, one or more of the first sensors 340 may be used to create a 4D model comprising the 3D surface of the patient's head and a 1D timeline. This 4D model is assigned to MR and/or CT scans and is used throughout the whole clinical workflow. Accordingly, the 4D model may be used as a primary imaging guidance for radiotherapy so as to significantly reduce a demand of X-ray and/or MR images.

For better understanding, Figure 8 schematically shows the general use of the first sensor 340. As explained above, one or more first sensors 340 are arranged at the coil housing 310 facing the receiving space 313. The sensor 340 captures the surface of the patient's head and this data is processed by the data processing device 110 and/or recorded to the storage unit 115.

Referring again to Figure 7, the second sensor 350, besides the above patient identification, may also be used to detect the patient's heart rate, mental stress, sleep patterns or the like. This may be performed during patient training, preparing and treatment under LINAC. Compared with X-ray, the working principle of the second sensor 340 is noninvasive.

Also, the synchronizing device 190 may perform several tasks within the MR device 100. Accordingly, since the second sensor 350 is configured as a PPG sensor, the synchronizing device 190 controls the first light source 211 and/or the second light source 230 via the light controlling unit 140 to change the emitted light to a spectrum which has less e.g. red components, such as a blue light. In this way, the emitted light is changed for only a few milliseconds required by the second sensor 350 for measuring. In this short period of time, the patient's eye hardly or not at all perceives the change in color. Further, when the gradient amplifier is active during MRI, the synchronizing device 190 is used to temporarily, in a range of e.g. 100 to 200 milliseconds, disable one or more of the electronically based components of the head coil arrangement 300, such as the first and second sensors 340, 350, the acoustic transducer 360 etc. In this way, these electronic components do not generate electronic noise which may introduce artifacts on the MR images . For the same reason, the synchronizing device 190 temporarily disables the acoustic transducer 360.

The fiducial markers 330, which are also arranged inside the receiving space 313, allow to monitor the position of the head coil arrangement 300, the patient's head and the head-fixation structure 320 in real-time. By combining the 3D surface scanning of the patient's head from first sensor and anatomy data inside of patient's brain from MR scanner, the positions and dimensions of patient's head and internal brain can be determined. Therefore, ERE effects can be predicted and taken into account during treatment dose planning. In Addition, during treatment, the radiation beam can be adapted in real-time such that the beam will not go through the conductive coil wires of the RF coil 314 or other electronic components of the head coil arrangement 300. In this way, the electronics are less impacted by radiation.

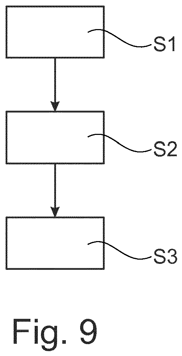

With reference to Figure 9, which shows a flowchart, an exemplary method of immobilizing a head within the MR device 100 will be described in the following. As noted above, the method can be modified according to the design and/or operation of the MR device 100.

First, in a step S1, the helmet-shaped coil housing 310 comprising the at least one conductor carrier 311, 312 extending in a distance to a head supporting plane and forming in between the receiving space 313 for a patient's head is provided. Then, in a step S2, the head-fixation structure 320, which is supported, via at least one leg 321, at the conductor carrier 311, 312 so as to at least partially protrude into the receiving space 313 and configured to be brought into noninvasive contact with the patient is provided. Then, in a step S3, movement of the patient's head relative to the coil housing 310 and/or the head-fixation structure 320 is monitored by utilizing one or more markers 330 and/or one or more sensors 340, 350 arranged at the coil housing 310 and/or the head-fixation structure 320.

### Reference signs

- 100: magnetic resonance device
- 110: data processing device
- 115: storage unit
- 120: MRI post processing unit
- 130: gradient amplifier
- 140: light controlling unit
- 150: LINAC interface
- 160: optical converter
- 170: MRI signal readout unit
- 180: MRI signal receiver
- 190: synchronizing device

- 200: magnetic resonance scanner
- 210: bore
- 211: first light source
- 220: support table
- 230: second light source

- 300: head coil arrangement
- 310: helmet-shaped coil housing
- 311: first conductor carrier
- 312: second conductor carrier
- 313: receiving space
- 314: RF coil

- 320: head-fixation structure
- 321: leg
- 322: bite-lock mouthpiece
- 323: chinrest
- 324: frontal bone holder
- 330: fiducial marker
- 340: first sensor (e.g. time-of-flight-sensor)
- 350: second sensor (e.g. photoplethysmogram-sensor)
- 360: acoustic transducer

- S1-S3: method steps

## Claims

1. A head coil arrangement (300) for a magnetic resonance device (100), comprising
- a helmet-shaped coil housing (310) comprising at least one conductor carrier (311, 312) extending in a distance to a head supporting plane and forming in between a receiving space (313) for a patient's head, and
- a head-fixation structure (320), which is supported, via at least one leg (321), at the conductor carrier (311, 312) so as to at least partially protrude into the receiving space (313) and configured to be brought into blunt contact with the patient,
wherein the head-fixation structure (320) is molded in one piece with the coil housing (310).

2. The head coil arrangement (300) according to claim 1, further comprising at least one first conductor carrier (311) and at least one second conductor carrier (312) which together span a conductor plane at least partially surrounding the receiving space (313).

3. The head coil arrangement (300) according to claim 1 or claim 2, wherein the head-fixation structure (320) is made from an electromagnetically inert material.

4. The head coil arrangement (300) according to any one of the preceding claims, wherein the head-fixation structure (320) comprises a bite-lock mouthpiece (322).

5. The head coil arrangement (300) according to any one of the preceding claims, wherein the head-fixation structure comprises a chinrest (323).

6. The head coil arrangement (300) according to any one of the preceding claims, wherein the head-fixation structure comprises a frontal bone holder (324).

7. The head coil arrangement (300) according to any one of the preceding claims, wherein both the head-fixation structure (320) and the coil housing (310) comprise at least one fiducial marker (330) arranged so as to be trackable within a magnetic resonance image.

8. The head coil arrangement (300) according to any one of the preceding claims, wherein the coil housing (310) comprises at least one first sensor (340) configured to measure a signal propagation time for determining a position of the patient relative to the coil housing (310).

9. The head coil arrangement (300) according to any one of the preceding claims, wherein the coil housing (310) comprises at least one second sensor (350) configured to measure a light absorption or the light remission on transillumination of a skin of the patient.

10. The head coil arrangement (300) according to any one of the preceding claims, further comprising at least one acoustic transducer (360) arranged at the head-fixation structure (320) and configured to send an acoustic signal via one or more cranial bones to an internal ear of the patient.

11. A magnetic resonance device (100), comprising
- a magnetic resonance scanner (200), and
- a head coil arrangement (300) according to any one of the preceding claims.

12. The magnetic resonance device (100) according to claim 11, further comprising a data processing device (110) configured to create a multi-dimensional model of a patient's head on basis of data provided by one or more sensors (340, 350) arranged at the head coil arrangement (300).

13. The magnetic resonance device (100) according to claim 12, wherein the model comprises three geometric dimensions extended by a fourth time-related dimension.

14. The magnetic resonance device (100) according to claim 12 or 13, further comprising a synchronizing device (120) communicatively connected to both the head coil arrangement (300) and the magnetic resonance scanner (200) and configured to temporarily put an electrical component (211, 340, 350, 360) of one of the magnetic resonance scanner (200) and the head coil arrangement (300) in a different operating state during a specific task performed with the other one of the magnetic resonance scanner (200) and the head coil arrangement (300).

15. The magnetic resonance device (100) according to claim 14, wherein the synchronizing device (120) is configured to temporarily change a light emitted by a light source (211) arranged in a bore (210) of the magnetic resonance scanner (200) while controlling a measurement via one or more optical sensors (340, 350) arranged at the head coil arrangement (300).

16. The magnetic resonance device (100) according to claim 14 or 15, wherein the synchronizing device (120) is configured to temporarily disable a power supply of one or more optical sensors (340, 350) arranged at the head coil arrangement (300) during imaging by the magnetic resonance scanner (200).

17. The magnetic resonance device (100) according to any one of claims 14 to 16, wherein the synchronizing device (120) is configured to temporarily disable a power supply of an acoustic transducer (360) of the head coil arrangement (300) during imaging by the magnetic resonance scanner (200).

18. Method of immobilizing a head within a magnetic resonance device (100), comprising:
- providing (S1) a helmet-shaped coil housing (310) comprising at least one conductor carrier (311, 312) extending in a distance to a head supporting plane and forming in between a receiving space (313) for a patient's head, and
- providing (S2) a head-fixation structure (320), which is supported, via at least one leg (321), at the conductor carrier (311, 312) so as to at least partially protrude into the receiving space (313) and configured to be brought into blunt contact with the patient, wherein the head-fixation structure (320) is molded in one piece with the coil housing (310),
- wherein a movement relative to the coil housing (310) and/or the head-fixation structure (320) is monitored (S3) by utilizing one or more markers (330) and/or one or more sensors (340, 350) arranged at the coil housing (310) and/or the head-fixation structure (320).

19. The method according to claim 18, further comprising:
- scanning a surface of the patient's head by utilizing the one or more sensors (340, 350),
- creating a multi-dimensional model of the patient's head on basis of data obtained by the scanning, and
- verifying (S5), on the basis of the model, if the position of the patient's head is correct.

20. The method according to claim 19, wherein, on the basis of the model, real-time treatment dose planning is performed.

## Patentansprüche

1. Kopfspulenanordnung (300) für ein Magnetresonanzgerät (100), umfassend
- ein helmförmiges Spulengehäuse (310) mit mindestens einem Leiterträger (311, 312), der sich in einem Abstand zu einer Kopfstützebene erstreckt und dazwischen einen Aufnahmeraum (313) für den Kopf eines Patienten bildet, und
- eine Kopffixierungsstruktur (320), die über mindestens ein Bein (321) an dem Leiterträger (311, 312) so abgestützt ist, dass sie mindestens teilweise in den Aufnahmeraum (313) hineinragt und so konfiguriert ist, dass sie in stumpfen Kontakt mit dem Patienten gebracht werden kann,
wobei die Kopffixierungsstruktur (320) in einem Stück mit dem Spulengehäuse (310) geformt ist.

2. Kopfspulenanordnung (300) nach Anspruch 1, ferner umfassend zumindest einen ersten Leiterträger (311) und zumindest einen zweiten Leiterträger (312), die zusammen eine Leiterebene aufspannen, die den Aufnahmeraum (313) zumindest teilweise umgibt.

3. Kopfspulenanordnung (300) nach Anspruch 1 oder Anspruch 2, wobei die Kopffixierungsstruktur (320) aus einem elektromagnetisch inerten Material hergestellt ist.

4. Kopfspulenanordnung (300) nach einem der vorstehenden Ansprüche, wobei die Kopffixierungsstruktur (320) ein Mundstück (322) mit Bisssicherung umfasst.

5. Kopfspulenanordnung (300) nach einem der vorstehenden Ansprüche, wobei die Kopffixierungsstruktur eine Kinnauflage (323) umfasst.

6. Kopfspulenanordnung (300) nach einem der vorstehenden Ansprüche, wobei die Kopffixierungsstruktur einen Stirnbeinhalter (324) umfasst.

7. Kopfspulenanordnung (300) nach einem der vorstehenden Ansprüche, wobei sowohl die Kopffixierungsstruktur (320) als auch das Spulengehäuse (310) mindestens eine AO:TE Referenzmarkierung (330) aufweisen, die so angeordnet ist, dass sie innerhalb eines Magnetresonanzbildes verfolgt werden kann.

8. Kopfspulenanordnung (300) nach einem der vorstehenden Ansprüche, wobei das Spulengehäuse (310) mindestens einen ersten Sensor (340) umfasst, der dazu konfiguriert ist, eine Signallaufzeit zur Bestimmung einer Position des Patienten relativ zum Spulengehäuse (310) zu messen.

9. Kopfspulenanordnung (300) nach einem der vorstehenden Ansprüche, wobei das Spulengehäuse (310) mindestens einen zweiten Sensor (350) umfasst, der dazu eingerichtet ist, eine Lichtabsorption oder die Lichtremission bei Durchleuchtung einer Haut des Patienten zu messen.

10. Kopfspulenanordnung (300) nach einem der vorstehenden Ansprüche, ferner umfassend mindestens einen akustischen Wandler (360), der an der Kopffixierungsstruktur (320) angeordnet und dazu eingerichtet ist, ein akustisches Signal über einen oder mehrere Schädelknochen an ein Innenohr des Patienten zu senden.

11. Magnetresonanzgerät (100), umfassend
- einen Magnetresonanztomographen (200), und
- eine Kopfspulenanordnung (300) nach einem der vorstehenden Ansprüche.

12. Magnetresonanzgerät (100) nach Anspruch 11, ferner umfassend eine Datenverarbeitungsvorrichtung (110), die dazu eingerichtet ist, ein mehrdimensionales Modell des Kopfes eines Patienten auf der Grundlage von Daten zu erzeugen, die von einem oder mehreren Sensoren (340, 350) geliefert werden, die an der Kopfspulenanordnung (300) angeordnet sind.

13. Magnetresonanzgerät (100) nach Anspruch 12, wobei das Modell drei geometrische Dimensionen umfasst, die um eine vierte zeitbezogene Dimension erweitert sind.

14. Magnetresonanzgerät (100) nach Anspruch 12 oder 13, ferner umfassend eine Synchronisationsvorrichtung (120), die kommunikativ sowohl mit der Kopfspulenanordnung (300) als auch mit dem Magnetresonanztomographen (200) verbunden und dazu eingerichtet ist, eine elektrische Komponente (211, 340, 350, 360) entweder des Magnetresonanztomographen (200) oder der Kopfspulenanordnung (300) während einer spezifischen Aufgabe, die mit dem anderen der beiden Komponenten Magnetresonanztomograph (200) und Kopfspulenanordnung (300) durchgeführt wird, vorübergehend in einen anderen Betriebszustand zu versetzen.

15. Magnetresonanzgerät (100) nach Anspruch 14, wobei die Synchronisierungsvorrichtung (120) dazu eingerichtet ist, vorübergehend ein Licht zu ändern, das von einer Lichtquelle (211) emittiert wird, die in einer Bohrung (210) des Magnetresonanztomographen (200) angeordnet ist, während sie eine Messung über einen oder mehrere optische Sensoren (340, 350) steuert, die an der Kopfspulenanordnung (300) angeordnet sind.

16. Magnetresonanzgerät (100) nach Anspruch 14 oder 15, wobei die Synchronisierungsvorrichtung (120) dazu eingerichtet ist, die Stromversorgung eines oder mehrerer optischer Sensoren (340, 350), die an der Kopfspulenanordnung (300) angeordnet sind, während der Bildgebung durch den Magnetresonanztomographen (200) vorübergehend zu deaktivieren.

17. Magnetresonanzgerät (100) nach einem der Ansprüche 14 bis 16, wobei die Synchronisierungsvorrichtung (120) dazu eingerichtet ist, die Stromversorgung eines akustischen Wandlers (360) der Kopfspulenanordnung (300) während der Bildgebung durch den Magnetresonanz-Scanner (200) vorübergehend zu deaktivieren.

18. Verfahren zum Immobilisieren eines Kopfes innerhalb eines Magnetresonanzgerätes (100), umfassend:
- Bereitstellen (S1) eines helmförmigen Spulengehäuses (310) mit mindestens einem Leiterträger (311, 312), der sich in einem Abstand zu einer Kopfstützebene erstreckt und dazwischen einen Aufnahmeraum (313) für den Kopf eines Patienten bildet, und
- Bereitstellen (S2) einer Kopffixierungsstruktur (320), die über mindestens ein Bein (321) am Leiterträger (311, 312) so abgestützt ist, dass sie mindestens teilweise in den Aufnahmeraum (313) hineinragt und dazu eingerichtet ist, in stumpfen Kontakt mit dem Patienten gebracht zu werden, wobei die Kopffixierungsstruktur (320) in einem Stück mit dem Spulengehäuse (310) geformt ist,
wobei eine Bewegung relativ zu dem Spulengehäuse (310) und/oder der Kopffixierungsstruktur (320) unter Verwendung von einem oder mehreren Markern (330) und/oder einem oder mehreren Sensoren (340, 350), die an dem Spulengehäuse (310) und/oder der Kopffixierungsstruktur (320) angeordnet sind, überwacht wird (S3).

19. Verfahren nach Anspruch 18, weiter umfassend:
- das Abtasten einer Oberfläche des Kopfes des Patienten unter Verwendung eines oder mehrerer Sensoren (340, 350),
- Erstellung eines mehrdimensionalen Modells des Kopfes des Patienten auf der Grundlage der durch das Scannen gewonnenen Daten, und
- Überprüfen (S5) anhand des Modells, ob die Kopfposition des Patienten korrekt ist.

20. Verfahren nach Anspruch 19, wobei auf der Grundlage des Modells eine Behandlungsdosisplanung in Echtzeit durchgeführt wird.

## Revendications

1. Agencement de bobine de tête (300) pour dispositif de résonance magnétique (100), comprenant :
- un logement de bobine en forme de casque (310) comprenant au moins un support de conducteur (311, 312) s'étendant à distance d'un plan de support de tête et formant au milieu un espace de réception (313) pour la tête d'un patient, et
- une structure de fixation de tête (320) qui est supportée, par l'intermédiaire d'au moins une jambe (321), sur le support de conducteur (311, 312), de manière à faire saillie au moins partiellement dans l'espace de réception (313) et conçue pour être mise en contact non pénétrant avec le patient,
dans lequel la structure de fixation de tête (320) est moulée d'une seule pièce avec le logement de bobine (310).

2. Agencement de bobine de tête (300) selon la revendication 1, comprenant en outre au moins un premier support de conducteur (311) et au moins un second support de conducteur (312) qui couvrent ensemble un plan de conducteur entourant au moins partiellement l'espace de réception (313).

3. Agencement de bobine de tête (300) selon la revendication 1 ou la revendication 2, dans lequel la structure de fixation de tête (320) est constituée d'un matériau électromagnétiquement inerte.

4. Agencement de bobine de tête (300) selon l'une quelconque des revendications précédentes, dans lequel la structure de fixation de tête (320) comprend un embout buccal formant bloc de morsure (322).

5. Agencement de bobine de tête (300) selon l'une quelconque des revendications précédentes, dans lequel la structure de fixation de tête comprend une mentonnière (323).

6. Agencement de bobine de tête (300) selon l'une quelconque des revendications précédentes, dans lequel la structure de fixation de tête comprend un élément de support d'os frontal (324).

7. Agencement de bobine de tête (300) selon l'une quelconque des revendications précédentes, dans lequel la structure de fixation de tête (320) et le logement de bobine (310) comprennent tous deux au moins une marque de repère (330) disposée de manière à pouvoir être suivie au sein d'une image de résonance magnétique.

8. Agencement de bobine de tête (300) selon l'une quelconque des revendications précédentes, dans lequel le logement de bobine (310) comprend au moins un premier capteur (340) conçu pour mesurer un temps de propagation de signal afin de déterminer une position du patient par rapport au logement de bobine (310).

9. Agencement de bobine de tête (300) selon l'une quelconque des revendications précédentes, dans lequel le logement de bobine (310) comprend au moins un second capteur (350) conçu pour mesurer une absorption de la lumière ou la rémission de la lumière lors de la transillumination de la peau du patient.

10. Agencement de bobine de tête (300) selon l'une quelconque des revendications précédentes, comprenant en outre au moins un transducteur acoustique (360) placé sur la structure de fixation de tête (320) et conçu pour envoyer un signal acoustique par l'intermédiaire d'un ou de plusieurs os crânien(s) à une oreille interne du patient.

11. Dispositif de résonance magnétique (100) comprenant :
- un appareil d'imagerie par résonance magnétique (200), et
- un agencement de bobine de tête (300) selon l'une quelconque des revendications précédentes.

12. Dispositif de résonance magnétique (100) selon la revendication 11, comprenant en outre un dispositif de traitement de données (110) conçu pour créer un modèle multidimensionnel de la tête d'un patient sur la base de données fournies par un ou plusieurs capteurs (340, 350) placé(s) sur l'agencement de bobine de tête (300).

13. Dispositif de résonance magnétique (100) selon la revendication 12, dans lequel le modèle comprend trois dimensions géométriques auxquelles s'ajoute une quatrième dimension se rapportant au temps.

14. Dispositif de résonance magnétique (100) selon la revendication 12 ou 13, comprenant en outre un dispositif de synchronisation (120) connecté à la fois à l'agencement de bobine de tête (300) et à l'appareil d'imagerie par résonance magnétique (200) de manière à pouvoir communiquer avec eux, et conçu pour mettre temporairement un composant électrique (211, 340, 350, 360) de l'un de l'appareil d'imagerie par résonance magnétique (200) ou de l'agencement de bobine de tête (300) dans un état de fonctionnement différent au cours d'une tâche spécifique accomplie par l'autre de l'appareil d'imagerie par résonance magnétique (200) ou de l'agencement de bobine de tête (300).

15. Dispositif de résonance magnétique (100) selon la revendication 14, dans lequel le dispositif de synchronisation (120) est conçu pour modifier temporairement une lumière émise par une source de lumière (211) placée dans un alésage (210) de l'appareil d'imagerie par résonance magnétique (200) tout en commandant une mesure par le biais d'un ou de plusieurs capteur(s) optique(s) (340, 350) placé(s) sur l'agencement de bobine de tête (300).

16. Dispositif de résonance magnétique (100) selon la revendication 14 ou 15, dans lequel le dispositif de synchronisation (120) est conçu pour désactiver temporairement une alimentation électrique d'un ou de plusieurs capteur(s) optique(s) (340, 350) placé(s) sur l'agencement de bobine de tête (300) au cours de l'imagerie au moyen de l'appareil d'imagerie par résonance magnétique (200).

17. Dispositif de résonance magnétique (100) selon l'une quelconque des revendications 14 à 16, dans lequel le dispositif de synchronisation (120) est conçu pour désactiver temporairement une alimentation électrique d'un transducteur acoustique (360) de l'agencement de bobine de tête (300) au cours de l'imagerie au moyen de l'appareil d'imagerie par résonance magnétique (200).

18. Procédé d'immobilisation d'une tête à l'intérieur d'un dispositif de résonance magnétique (100), comprenant les étapes suivantes :
- utiliser (S1) un logement de bobine en forme de casque (310) comprenant au moins un support de conducteur (311, 312) s'étendant à distance d'un plan de support de tête et formant au milieu un espace de réception (313) pour la tête d'un patient, et
- utiliser (S2) une structure de fixation de tête (320) qui est supportée, par l'intermédiaire d'au moins une jambe (321), sur le support de conducteur (311, 312), de manière à faire saillie au moins partiellement dans l'espace de réception (313) et conçue pour être mise en contact non pénétrant avec le patient, dans lequel la structure de fixation de tête (320) est moulée d'une seule pièce avec le logement de bobine (310),
- dans lequel un mouvement par rapport au logement de bobine (310) et/ou à la structure de fixation de tête (320) est surveillé (S3) au moyen d'une ou de plusieurs marque(s) (330) et/ou d'un ou de plusieurs capteur(s) (340, 350) placé (e) (s) sur le logement de bobine (310) et/ou sur la structure de fixation de tête (320).

19. Procédé selon la revendication 18, comprenant en outre les étapes suivantes :
- balayer une surface de la tête du patient en utilisant le ou les capteur(s) (340, 350),
- créer un modèle multidimensionnel de la tête du patient sur la base de données obtenues par le balayage, et
- vérifier (S5), sur la base du modèle, si la position de la tête du patient est correcte.

20. Procédé selon la revendication 19, dans lequel, sur la base du modèle, une planification de la dose de traitement en temps réel est réalisée.
